# EUROPEAN PATENT APPLICATION

(11) **EP 4 821 932 A1**
(43) Date of publication of application: **14.10.2026**
(21) Application number: 24901086.9
(22) Date of filing: 05.12.2024
(51) Int. Cl.: C07D 487/04, A61K 31/519

(54) **PHOSPHATE SALT OF 1-((3S,4R)-3-((2-((1-ETHYL-1H-PYRAZOL-4-YL)AMINO)-7H-PYRROLO[2,3-D]PYRIMIDIN-4-YL)OXY)-4-FLUOROPIPERIDIN-1-YL)PROP-2-EN-1-ONE, CRYSTALLINE FORM THEREOF, AND METHOD FOR PREPARATION THEREOF**

(30) Priority: 05.12.2023 KR 20230174736; 05.12.2023 KR 20230174738; 16.01.2024 KR 20240006933; 18.11.2024 KR 20240164590
(71) Applicant: Daewoong Pharmaceutical Co., Ltd., Hwaseong-si, Gyenoggi-do 18623 (KR)
(72) Inventor: PARK, Joon Seok, Gyeonggi-do 17000 (KR); HYUN, Hyae Jung, Seongnam-si, Gyeonggi-do 13568 (KR); LEE, Joon-Hwan, Suwon-si, Gyeonggi-do 16693 (KR); JUN, Sun Ah, Yongin-si, Gyeonggi-do 17027 (KR); KIM, Namyoun, Yongin-si, Gyeonggi-do 17002 (KR); KIM, Wonsun, Hwaseong-si, Gyeonggi-do 18430 (KR); KIM, Cheolwoong, Suwon-si, Gyeonggi-do 16684 (KR)
(74) Representative: Germain Maureau
(86) International application number: PCT/KR2024/019814
(87) International publication number: WO 2025/121900

(57) **Abstract**

The present disclosure relates to a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, a crystalline form thereof and a method for preparation thereof. The phosphate salt and crystalline form have low hygroscopicity compared to the free base and is improved in stability and solubility, thereby being suitable for pharmaceutical use.

In addition, the method for preparing the phosphate salt and crystalline form according to the present disclosure has the advantage that mass production is made possible and the safety of the preparation process is improved by using safer materials than the reactants used in conventional preparation methods.

## Description

### [TECHNICAL FIELD]

### CROSS-REFERENCE TO RELATED APPLICATION(S)

This application claims priority to and the benefit of Korean Patent Application No. 10-2023-0174736, filed on December 5, 2023, Korean Patent Application No. 10-2023-0174738, filed on December 5, 2023, Korean Patent Application No. 10-2024-0006933, filed on January 16, 2024, and Korean Patent Application No. 10-2024-0164590, filed on November 18, 2024, with the Korean Intellectual Property Office, the disclosures of which are incorporated herein by reference in their entirety.

The present disclosure relates to a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, a crystalline form thereof and a method for preparation thereof.

### [BACKGROUND]

The selection of pharmaceutically acceptable salts and their polymorphs is a very important step in the process of research and development of new drugs. This is because certain salt forms and their polymorphs can have a significant impact on the production process development of drug raw materials, and the design and formulation of the finished drug product.

Specifically, the type of salts and their polymorphs may affect recrystallization yield, process speed, and purity in the final stages of producing the drug raw material, that is, in the recrystallization and purification process. Particularly, the speed in the crystallization process may vary depending on the size or shape of crystals, and thus, the selection of salts and polymorphs is also important from the viewpoint of the productivity and production cost of the drug raw material.

Additionally, the addition of salts to certain drugs can be a means of changing the physiochemical and biological properties without modifying the chemical structure of the drug. This is because in pharmaceutical aspects, physicochemical properties such as hygroscopicity, stability, solubility, flowability of particles and dissolution rate are affected by the form of salts and their polymorphs. Thus, the form of salts and their polymorphs are factors that determine the production process, production and storage conditions, shelf life and the like of the finished drug product.

Therefore, there is a need for continuous research on the selection of salts and polymorphs that can exhibit better stability and better bioavailability in the preparation of specific drug formulations.

Meanwhile, the present inventors have found 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one as a novel active pharmaceutical ingredient having excellent inhibitory activity as a kinase inhibitor of both JAK3 and BTK.

Therefore, the present inventors have conducted intensive research on the selection of pharmaceutically acceptable salts and their polymorphs that can improve the solubility and stability of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, and as a result, found that when preparing into a phosphate salt form, it can be obtained as a crystalline structure, and can improve solubility, crystallinity, and stability under severe conditions, and completed the present disclosure.

### [DETAILED DESCRIPTION OF THE INVENTION]

### [Technical Problem]

It is an object of the present disclosure to provide a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

It is another object of the present disclosure to provide a method for preparing a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

It is another object of the present disclosure to provide a crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

It is yet another object of the present disclosure to provide a pharmaceutical composition for preventing or treating inflammatory diseases, autoimmune disease, proliferative disease, hyperproliferative disease, immunologically mediated disease, cancer, or tumor, comprising at least one selected from the group consisting of a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, and a crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

It is further yet another object of the present disclosure to provide a novel intermediate compound obtained in the process of preparing a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

### [Technical Solution]

In order to achieve the above objects, there is provided a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

Also, there is provided a method for preparing a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, which is a compound represented by the following Chemical Formula 1. More specifically, the preparation method comprises the following steps, wherein at least one of the following steps 1 to 4 further comprises a crystallization step:
(step 1) reacting a compound represented by the following Chemical Formula 1-1 and a compound represented by the following Chemical Formula 1-2 in the presence of a base to prepare a compound represented by the following Chemical Formula 1-3;
(step 2) reacting a compound represented by the following Chemical Formula 1-3 and a compound represented by the following Chemical Formula 1-4 in the presence of a palladium catalyst and a base to prepare a compound represented by the following Chemical Formula 1-5;
(step 3) reacting a compound represented by the following Chemical Formula 1-5 with an acid to prepare a compound represented by the following Chemical Formula 1-6;
(step 4) reacting a compound represented by the following Chemical Formula 1-6 and a compound represented by the following Chemical Formula 1-7 in the presence of a base to prepare a compound represented by the following Chemical Formula 1; and
(step 5) reacting a compound represented by the following Chemical Formula 1 with phosphoric acid to prepare a phosphate salt of a compound represented by the following Chemical Formula 1. 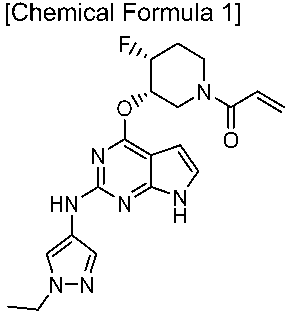 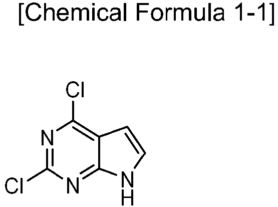 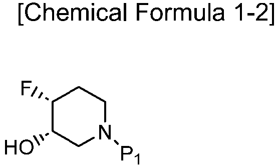 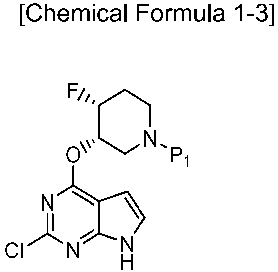 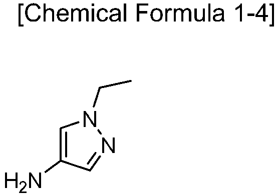 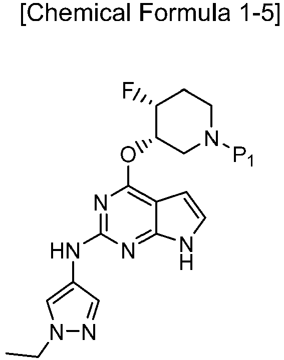 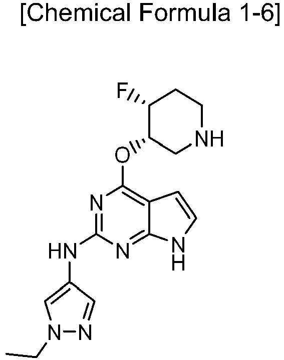 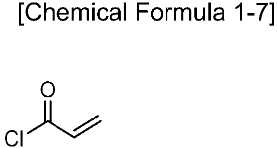 wherein in Chemical Formulas 1-1 to 1-7,
   P₁ means a protecting group.

Further provided herein is a crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

Further, there is provided a method for preparing a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, comprising a compound represented by the following Chemical Formula 1-5 as an intermediate: 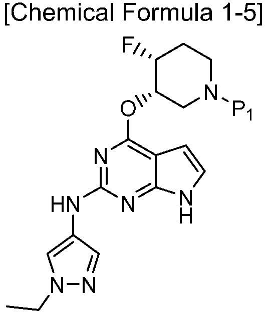 wherein in Chemical Formula 1-5,
P₁ means a protecting group.

Further, there is provided a compound represented by the following Chemical Formula 1-5: 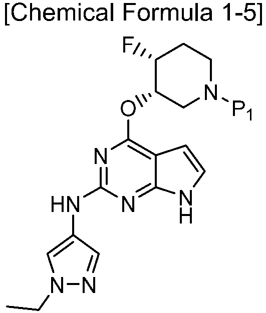 wherein in Chemical Formula 1-5,
P₁ means a protecting group.

Meanwhile, 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, which is a novel active pharmaceutical material, is represented by the following Chemical Formula 1:

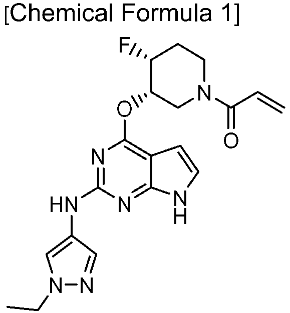

The 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one and pharmaceutically acceptable salts thereof can be favorably used for preventing or treating diseases against which kinase inhibitory action is efficacious. Therefore, the 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one and pharmaceutically acceptable salts thereof can be usefully applied to the prevention or treatment of inflammatory diseases, autoimmune disease, proliferative disease, hyperproliferative disease, immunologically mediated disease, cancer, or tumor.

Wherein, the pharmaceutically acceptable salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one is a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one. The phosphate salt can be prepared by reacting a free base of -((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one and a phosphoric acid to be added in a specific ratio.

Also, the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one may show a crystalline form. The phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one can be prepared by various crystallization methods such as evaporative crystallization, drawing-out crystallization, reaction crystallization, solvent-mediated polymorphism, and solid-state polymorphism, which are selected according to the thermodynamic and dynamic properties of the salt. Accordingly, the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one can be prepared in a crystalline form in itself.

Wherein, the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one or a crystalline form of the phosphate salt can be confirmed through X-ray powder diffraction analysis. Specifically, the phosphate salt or crystalline form can be distinguished through a diffraction angle (2θ) showing characteristic peaks in an X-ray powder diffraction pattern and the intensity of the peaks according to each diffraction angle (2θ ). Here, the diffraction angle (2θ) may be varied by ±0.2°, or preferably ±0.1°, depending on various factors such as the production technique of the sample for measurement, the procedure for fixing the sample for measurement, and the measurement instrument.

Further, the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one or a crystalline form of the phosphate salt can also be distinguished through the endothermic onset temperature and the endothermic temperature showing the maximum endothermic peak in differential scanning calorimetry analysis, respectively. Here, the temperature can be varied by ±3°C, preferably ±2°C±, more preferably ±1°C, depending on various factors such as the production technique of the sample for measurement, the measuring equipment, and the rate of temperature change.

Now, the present disclosure will be described in detail.

### [Phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one]

The free base (free form) of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one is known to have poor stability. Therefore, the present inventors have conducted intensive research on phosphoric acid in order to improve the stability of these compounds, and as a result, confirmed that when the compound and phosphoric acid are combined at a specific equivalent ratio, the stability and crystallinity can be improved.

The phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one may have three or more peaks selected from the group consisting of diffraction angles (2θ±0.2°) of 3.9°, 7.8°, 8.5°, 9.2°, 10.0°, 10.2°, 12.8 °, 14.4°, 15.8°, 16.0°, 20.8°, 21.2°, 21.6°, 22.3° and 25.5° in an X-ray powder diffraction pattern irradiated by Cu light source. Since the phosphate salt is prepared in both a free base and a crystalline form, an X-ray powder diffraction pattern is observed.

Also, the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one may have an endothermic onset temperature of 106.52 ± 1.0 °C and shows a maximum endothermic peak at the endothermic temperature of 123.8 ± 1.0 °C in differential scanning calorimetry analysis.

Meanwhile, the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one is prepared through the following Reaction Scheme 1: 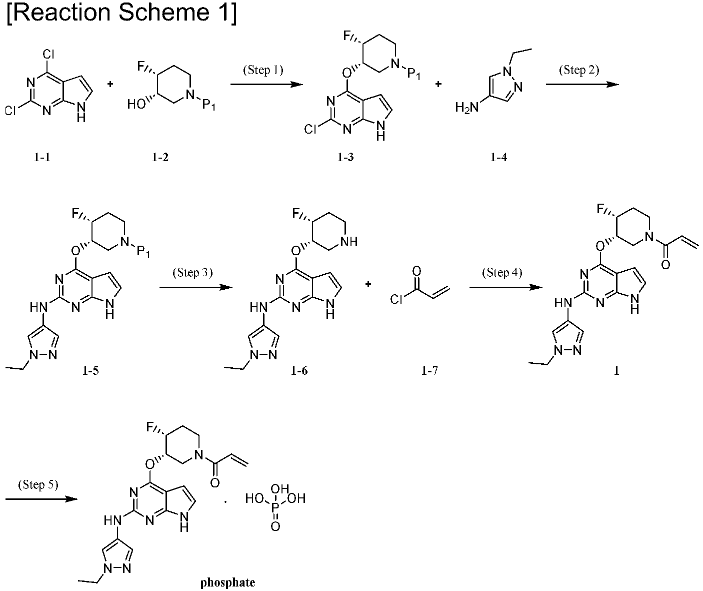 wherein in Reaction Scheme 1, P₁ means a protecting group.

More specifically, P₁ may be any one protecting group selected from the group consisting of tert-butoxycarbonyl(Boc), carbobenzyloxy(Cbz), para-methoxybenzylcarbonyl(Moz), 9-fluorenylmethyloxycarbonyl(Fmoc), acetyl(Ac), benzoyl(Bz), benzyl(Bn), and para-methoxybenzyl(PMB).

In this case, in terms of ease of production and yield, P₁ is preferably tert-butoxycarbonyl (Boc).

The preparation method does not include a separate substitution reaction of a protective group, and therefore the process steps are shortened compared with a conventional preparation method, and also does not go through a purification step by a column, and therefore is improved in yield and/or purity of the final product. Further, the preparation method involves crystallization purification in at least one or more steps, which is advantageous for mass production. In addition, the preparation method does not use the explosive strong bases (NaH, etc.) that were previously used, thereby ensuring safety during the process, and the reaction temperatures in some steps are lower than before, thereby being advantageous for application to general production plants and having economic effects.

Meanwhile, each step described below may further comprise a purification step after the reaction in order to prepare a compound with high purity and yield. In one example of the purification method, extraction using a difference in solubility, adsorption using activated carbon and/or silica-based materials, filtration, precipitation, crystallization, etc. may be used. However, the purification method does not use column chromatography, which is not suitable for mass production. Preferably, the purification step may be carried out as described in each step.

In addition, each step may further comprise a crystallization step using a crystallization solvent as the final step. The crystallization step may be carried out after the purification step if the purification step is carried out, and it may be carried out after the reaction if the purification step is not carried out. By further including the crystallization step, mass production is made possible. The crystallization solvent may be used according to the product of each step, but is not particularly limited. Preferably, the crystallization step may be carried out as described in each step.

### (Step 1)

The step 1 is a step of reacting the compound represented by Chemical Formula 1-1 and the compound represented by Chemical Formula 1-2 in the presence of a base to prepare a compound represented by Chemical Formula 1-3. The reaction is a nucleophilic aromatic substitution reaction, which is carried out in the presence of a base to make the added compound represented by Chemical Formula 1-2 into a nucleophile.

In the step 1, the compound represented by Chemical Formula 1-2 can be used in an amount of 0.1 to 10 equivalents based on 1 equivalent of the compound represented by Chemical Formula 1-1. Specifically, the compound represented by Chemical Formula 1-2 can be used in an amount of 0.2 to 5 equivalents, 0.3 to 3 equivalents, 0.5 to 2 equivalents, 0.7 to 1.5 equivalents, 1 to 1.5 equivalents, 1 to 1.3 equivalents, 1 to 1.05 equivalents, or 1.05 equivalents, based on 1 equivalent of the compound represented by Chemical Formula 1-1.

Further, in the step 1, the base may be at least one selected from the group consisting of potassium tert-butoxide, sodium tert-butoxide, potassium methoxide, sodium methoxide, potassium tert-pentoxide, sodium tert-pentoxide, triethylamine, diisopropylethylamine, sodium hydroxide, potassium hydroxide, lithium hydroxide, potassium carbonate, calcium carbonate, sodium carbonate, potassium hydrogen carbonate, sodium hydrogen carbonate and cesium carbonate. Among these bases, potassium tert-butoxide is preferably used in terms of reaction speed and yield. The base used is sufficient only by a non-explosive base, safety is secured, which is advantageous for industrialization.

And, the base can be used in an amount of 0.1 to 10 equivalents based on 1 equivalent of the compound represented by Chemical Formula 1-1. Preferably, the base can be used in an amount of 0.5 to 5 equivalents, 1 to 5 equivalents, 2 to 5 equivalents, 2 to 3 equivalents, or 3 equivalents, based on 1 equivalent of the compound represented by Chemical Formula 1-1. If the base is used in an excessively small amount, there is a risk that the reaction time is prolonged, and even if the base is used in an excessively large amount, there is no difference in the reaction time, and so it is preferable to use the base in the above-mentioned range.

In the step 1, the reaction may be carried out in one or more organic solvents selected from the group consisting of tetrahydrofuran(THF), 1,4-dioxane, methanol, ethanol, isopropanol, 1-butanol, 2-butanol, acetone, methyl ethyl ketone, ethyl acetate, methyl tert-butyl ether, acetonitrile, toluene, heptane, 3-methyltetrahydrofuran, dichloromethane, methyl isobutyl ketone, dimethyl formamide(DMF), dimethyl acetamide(DMAC), and dimethyl sulfoxide(DMSO). As an example, one type of the solvent may be used, or a mixed solvent of two or more types may be used. Preferably, 1,4-dioxane may be used as the organic solvent. 1,4-Dioxane has the advantage that it can produce products of higher purity than other solvents and it is easy to separate layers during extraction. Other solvents have the disadvantage that they produce relatively large amounts of impurities or have slow reaction rates.

Further, the organic solvent may be used in an amount of 5 to 30 times (mL/g), more specifically, 5 to 15 times (mL/g), based on the weight of the compound represented by Chemical Formula 1-1.

Further, the reaction can be carried out at 30 to 60 °C for 2 to 10 hours. If the reaction is carried out under temperature conditions lower than the above-mentioned range and/or for a short reaction time, the reaction may not proceed sufficiently, which may lead to the deterioration of a production yield. In addition, even if the reaction is carried out under temperature conditions higher than the above-mentioned range and/or for a long reaction time, the production yield is not substantially increased, which is not desirable in terms of process cost.

Meanwhile, after the reaction of the step 1 is completed, the method may further comprise a purification step of extracting the reaction product using ethyl acetate and/or water. Specifically, the reaction product may be extracted using ethyl acetate and water. That is, the compound represented by Chemical Formula 1-3 may be extracted using ethyl acetate and water after the completion of the reaction. The extraction using ethyl acetate and water may be carried out by a method in which the mixture containing the reaction product and each solvent is stirred for 30 minutes or more, allowed to stand for 30 minutes or more and then separated into layers, so that an aqueous layer (lower layer) is discarded and an organic layer (upper layer) is taken. The extraction using ethyl acetate and water may be repeated one or more times. In the case of such an extraction, impurities having a relative retention time of less than 1.0 as separated by high performance liquid chromatography(HPLC) can be effectively removed by the aqueous layer, compared with the case of extraction using a methylene chloride/water mixed solvent.

In addition, the step 1 may further comprise a step of filtering the extracted mixture, followed by vacuum concentration and/or azeotropic concentration. This vacuum concentration and/or azeotropic concentration step is preferably carried out after the extraction step with ethyl acetate and/or water.

In addition, the step 1 may further include a step of crystallizing a reaction product in order to purify the compound represented by Chemical Formula 1-3. Such a crystallization step is preferably carried out after the extraction and purification step. In other words, the step 1 can be carried out by a step in which, after the extraction and purification step, a crystallization solvent is added to the concentrated product and stirred at a temperature of about 20 to 50 °C for 30 minutes to 4 hours to prepare the compound represented by Chemical Formula 1-3.

When a reaction product is prepared by crystallization using a crystallization in this way, unlike column chromatography in which purification is carried out using a silica column, it is possible to apply to industrialization, which is advantageous in terms of industrial mass production, and has the advantage of being able to obtain a high yield.

Specifically, the crystallization step can be carried out by crystallization using water as a crystallization solvent. The crystallization step can be carried out by repeating the crystallization process one or more times, which proceeds in the order of 'adding the crystallization solvent and then stirring'. In this manner, when the crystallization process is repeated while adding the solvent multiple times instead of adding the solvent at once, it is possible to obtain even the crystals that adhere to the wall surface during the solvent addition process, thereby preventing a decrease in yield, effectively removing by-products remaining after the reaction, and crystallizing an uncrystallized product through repeated crystallization processes. Thereby, compared with a process of purifying in a slurry form using a solvent such as ethyl acetate, a high-purity compound can be obtained in a high yield.

The crystallization solvent used in one crystallization process may be used in an amount of volume(mL/g) of 3 to 20 times, more specifically, in an amount of volume(mL/g) of 3 to 10 times, relative to the weight of the compound represented by Chemical Formula 1-1.

One crystallization process may be carried out by stirring at a temperature range of 5 to 30 °C, preferably at room temperature (20 to 25 °C) for 10 minutes to 2 hours. The process time can be appropriately adjusted, for example, by increasing it when the number of repetitions of the crystallization process is small, and decreasing it when the number of repetitions is large.

That is, when all the steps of purifying and crystallizing a reaction product of the step 1 are carried out to prepare the compound represented by Chemical Formula 1-3, the step 1 may include the following steps, but is not limited to these steps:
a step of reacting the compound represented by Chemical Formula 1-1 with the compound represented by Chemical Formula 1-2 in the presence of a base;
a step of adding ethyl acetate and water to discard the aqueous layer (lower layer) and separate and extract the organic layer (upper layer), and step of vacuum concentrating and/or azeotropically concentrating the purified mixture; and
a step of crystallizing the concentrate.

After purifying the compound represented by Chemical Formula 1-3, the compound may be dried at 40 to 60 °C for 10 to 14 hours to reduce the moisture content contained in the compound represented by Chemical Formula 1-3. More specifically, if the moisture content of the compound represented by Chemical Formula 1-3 is significantly reduced by drying at 50 to 60 °C, the conversion rate of the next step (i.e., step 2 below) can be increased.

### (Step 2 and Step 3)

The step 2 is a step of reacting the compound represented by Chemical Formula 1-3 and the compound represented by Chemical Formula 1-4 in the presence of a palladium catalyst and a base to prepare the compound represented by Chemical Formula 1-5, wherein a Buchwald-Hartwig amination reaction is carried out. The Buchwald-Hartwig amination reaction is a reaction in which a carbon and nitrogen bond is formed through a palladium catalyzed coupling reaction of an amine and an aryl halide. In addition, the step 3 is a step of reacting the compound represented by Chemical Formula 1-5 with an acid to prepare the compound represented by Chemical Formula 1-6, which is a step for removing the protecting group P₁ contained in the Chemical Formula 1-5.

The concentrated residual liquid containing the compound represented by Chemical Formula 1-5 produced in the step 2 can be used as it is in the next step without a separate purification process to ensure a stable reaction without a decrease in yield or purity, etc. Thus, in consideration of the economic effect caused by omitting the purification step and the ease and convenience of the process, the concentrated residual liquid of the step 2 can be used as it is to configure the step 2 and step 3 in-situ.

In the step 2, the compound represented by Chemical Formula 1-4 can be used in an amount of 0.1 to 10 equivalents based on 1 equivalent of the compound represented by Chemical Formula 1-3. Specifically, the compound represented by Chemical Formula 1-4 can be used in an amount of 0.2 to 5 equivalents, 0.5 to 5 equivalents, 0.5 to 3 equivalents, 0.5 to 2 equivalents, 0.5 to 1.5 equivalents, 1 to 1.5 equivalents, or 1.5 equivalents, based on 1 equivalent of the compound represented by Chemical Formula 1-3.

In the step 2, the palladium catalyst may be a palladium(0) catalyst, where the valence of palladium(Pd) in the compound is zero, or a palladium(II) catalyst, where the valence is +2. For example, the palladium catalyst may be at least one selected from the group consisting of tris(dibenzylideneacetone)dipalladium(0), tetrakis(triphenylphosphine)palladium(0), bis[tris(2-methylphenyl)phosphine]palladium, palladium(II)acetate, 1,1-(bis(diphenylphosphino)ferrocene)palladium(II)dichloride, and palladium(II)chloride.

Further, in the step 2, the base may be at least one selected from the group consisting of potassium tert-butoxide, sodium tert-butoxide, potassium methoxide, sodium methoxide, potassium tert-pentoxide, sodium tert-pentoxide, triethylamine, diisopropylethylamine, sodium hydroxide, potassium hydroxide, lithium hydroxide, potassium carbonate, calcium carbonate, sodium carbonate, potassium hydrogen carbonate, sodium hydrogen carbonate and cesium carbonate. The base used is sufficient only by a non-explosive base, safety is secured, which is advantageous for industrialization.

Further, the reaction may be carried out in the presence of a phosphine-based compound together with the palladium catalyst and the base. As the phosphine-based compound, at least one selected from the group consisting of 2,2'-bis(diphenylphosphino)-1,1'-binaphthyl, 4,5-bis(diphenylphosphino)-9,9-dimethylxanthene, 2-(dicyclohexylphosphino)-3,6-dimethoxy-2,4'6'-triisopropyl-1,1'-biphenyl, 2-di-tert-butylphosphino-2',4',6'-triisopropylbiphenyl and dicyclohexylphosphino-2',6'-diisopropoxybiphenyl may be used.

Preferably, in the step 2, the base may be used in an amount of 0.1 to 10 equivalents based on 1 equivalent of the compound represented by Chemical Formula 1-3. Preferably, the base may be used in an amount of 0.5 to 5 equivalents, 1 to 5 equivalents, 2 to 5 equivalents, 3 to 5 equivalents, or 3 equivalents, based on 1 equivalent of the compound represented by Chemical Formula 1-3. If the base is used in an excessively small amount, there is a risk that the reaction time is prolonged, and even if the base is used in an excessively large amount, there is no difference in the reaction time, and thus the base is used preferably in the above-mentioned range.

As the reaction solvent in the step 2, a solvent that is inactive to the amine substitution reaction can be used. For example, the reaction may be carried out in one or more organic solvents selected from the group consisting of tetrahydrofuran(THF), 1,4-dioxane, sec-butyl alcohol, methanol, ethanol, isopropanol, 1-butanol, 2-butanol, tert-butyl alcohol, acetone, methyl ethyl ketone, ethyl acetate, methyl tert-butyl ether, acetonitrile, toluene, xylene, heptane, 3-methyltetrahydrofuran, dichloromethane, methyl isobutyl ketone, dimethyl formamide(DMF), dimethyl acetamide(DMAC), and dimethyl sulfoxide(DMSO). As an example, one type of the solvent may be used, or a mixed solvent of two or more types may be used, but sec-butyl alcohol is preferable in terms of reaction speed and yield.

The organic solvent may be used in an amount of volume(mL/g) of 0.5 to 10 times, specifically, in an amount of volume(mL/g) of 4 to 8 times, or 4 to 6 times, relative to the weight of the compound represented by Chemical Formula 1-3. If the solvent is excessive during the reaction of the step 2, there is a problem that the reaction does not proceed.

Further, the reaction may be carried out at a temperature of 80 to 100 °C for 1 to 10 hours. If the reaction is carried out under temperature conditions lower than the above-mentioned range and/or for a short reaction time, the reaction may not proceed sufficiently, which may lead to the deterioration of a production yield. In addition, even if the reaction is carried out under temperature conditions higher than the above-mentioned range and/or for a long reaction time, the production yield is not substantially increased, which is not preferable in terms of process cost. More specifically, the reaction can be carried out at a temperature of 85 to 95 °C or 88 to 92 °C for 1 to 6 hours, 1 to 4 hours, preferably 1 to 3 hours, and more preferably 1.5 to 2 hours. The reaction temperature is in a range far below the boiling point of the reaction solvent, which is significantly lower than conventional reaction temperatures, thereby making it easy to apply in the process and reducing time and costs.

Further, the reaction can be carried out in a commonly known reactor when using the combination of the above-mentioned palladium catalyst, base, and phosphine-based compound. Thereby, the step 2 can be applied as one step in the process for mass-producing the compound represented by Chemical Formula 1.

Meanwhile, after the reaction of the step 2 is completed, the method may further comprise a purification step of extracting the reaction product using ethyl acetate and water or using ethyl acetate and a sodium chloride aqueous solution. Specifically, the reaction product may be extracted by using ethyl acetate and water or using ethyl acetate and a sodium chloride aqueous solution or using both of the above methods. That is, the compound represented by Chemical Formula 1-5 may be extracted by using ethyl acetate and water or using ethyl acetate and a sodium chloride aqueous solution after the reaction is completed. The extraction using ethyl acetate and water or using ethyl acetate and a sodium chloride aqueous solution may be carried out by a method in which the mixture containing the reaction product and each solvent is stirred for 30 minutes or more, allowed stand for 30 minutes or more and then separated into layers, so that an aqueous layer (lower layer) is discarded and an organic layer (upper layer) is taken. The extraction using ethyl acetate and water may be repeated one or more times. In the case of such an extraction, impurities having a relative retention time of less than 1.0 as separated by high performance liquid chromatography(HPLC) can be effectively removed by the aqueous layer, compared with the case of extraction using a methylene chloride/water mixed solvent.

In addition, the step 2 may further comprise a step of vacuum concentrating and/or azeotropically concentrating the extracted mixture. The vacuum concentration and/or azeotropically concentrating steps are preferably carried out after the extraction step using the ethyl acetate and water; or the ethyl acetate and sodium chloride aqueous solution.

That is, when all the steps of purifying the reaction product of the step 2 are carried out to prepare the compound represented by Chemical Formula 1-5, the step 2 may include the following steps, but is not limited to these steps:
a step of reacting the compound represented by Chemical Formula 1-3 and the compound represented by Chemical Formula 1-4 in the presence of a palladium catalyst and a base;
a step of adding ethyl acetate and water, or ethyl acetate and an aqueous sodium chloride solution to discard the aqueous layer (lower layer) and separate and extract the organic layer (upper layer); and
a step of vacuum concentrating and/or azeotropically concentrating the purified mixture.

Meanwhile, after the reaction is completed, the process of step 2 and step 3 described below can be carried out in-situ in the same reaction vessel, without a process of purifying or separating the compound represented by Chemical Formula 1-5. In this manner, since the process of purifying and separating the compound in the step 2 is not required, the method for preparing the compound represented by Chemical Formula 1 including the step 2 may be advantageous in terms of industrial mass production of the compound.

The step 3 is a step of reacting the compound represented by Chemical Formula 1-5 with an acid to prepare the compound represented by Chemical Formula 1-6, which is a step of removing the protecting group P₁ contained in Chemical Formula 1-5.

As the acid used in the reaction of the step 3, at least one selected from the group consisting of hydrochloric acid, acetic acid, trichloroacetic acid, trifluoroacetic acid, bromic acid, sulfuric acid, nitric acid, and iodic acid may be used. Among these acids, it is preferable to use hydrochloric acid when considering the yield and cost. Here, concentrated hydrochloric acid(c-HCl) may be used as the hydrochloric acid. For example, concentrated hydrochloric acid with a concentration of about 30 to 40 wt.% can be used.

The acid can be used in an amount of volume (mL/g) of 1 to 10 times, more specifically, in an amount of volume (mL/g) of 2 to 5 times, relative to the weight of the compound represented by Chemical Formula 1-5.

The reaction solvent of the step 3 may be one or more organic solvents selected from the group consisting of ethyl acetate, dichloromethane, butyl acetate, trichloromethane, methanol, ethanol, isopropanol, 1-butanol, 2-butanol, acetone, methyl ethyl ketone, methyl tert-butyl ether, acetonitrile, toluene, heptane, tetrahydrofuran, 3-methyltetrahydrofuran, 1,4-dioxane, methyl isobutyl ketone, dimethylformamide(DMF), dimethylacetamide(DMAC), and dimethyl sulfoxide(DMSO). As an example, one type of the solvent may be used, or a mixed solvent of two or more types may be used.

The organic solvent may be used in an amount of volume (mL/g) of 5 to 30 times, more specifically, in an amount of volume (mL/g) of 5 to 15 times, or 5 to 6 times, relative to the weight of the compound represented by Chemical Formula 1-5.

Further, the reaction can be carried out at 0°C to 50°C for 1 hour to 10 hours, preferably 3 hours to 5 hours. The removal reaction of the tert-butoxycarbonyl group, which is a protecting group, corresponds to a reaction that easily occurs without additional heat treatment, and can proceed within the above temperature range. However, if the reaction time is less than 1 hour, the reaction may not proceed sufficiently, and if the reaction time exceeds 10 hours, the production yield is not substantially increased, and thus the above-mentioned reaction time is preferred.

Meanwhile, after the reaction of the step 3 is completed, the method may further comprise a purification step of extracting the reaction product. Preferably, the solvent used for the extraction is not limited, but as an example, the extraction step may extract the reaction product using dichloromethane and water or using ethyl acetate and water. That is, the compound represented by Chemical Formula 1-6 may be extracted using dichloromethane and water or using ethyl acetate and water or both of the above methods after the reaction is completed.

The extraction using dichloromethane and water can be carried out by a method in which the mixture containing the reaction product and each solvent is stirred for 30 minutes or more, allowed to stand for 30 minutes or more and then separated into layers, so that an organic layer (lower layer) is discarded, and an aqueous layer (upper layer) is taken. The extraction using ethyl acetate and water can be carried out by a method in which the mixture containing the reaction product and each solvent is stirred for 30 minutes or more, allowed to stand for 30 minutes or more and then separated into layers, so that an aqueous layer (lower layer) is discarded, and an organic layer (upper layer) is taken. The extraction using dichloromethane and water or the extraction using ethyl acetate and water may be repeated one or more times. Meanwhile, the extraction using ethyl acetate and water is preferably carried out in the presence of a base such as sodium hydroxide while maintaining the pH of the reaction product at 9 to 11.

In addition, the step 3 may further comprise a step of vacuum concentrating and/or azeotropically concentrating the extracted mixture. This vacuum concentration and/or azeotropic concentration steps are preferably carried out after the extraction step using dichloromethane and water; or ethyl acetate and water.

And, the step 3 may further comprise a step of crystallizing a reaction product in order to purify the compound represented by Chemical Formula 1-6. This crystallization step is preferably carried out after the extraction purification step. In other words, the step 3 may be carried out by a step of adding a crystallization solvent to the concentrated product after the extraction purification step and stirring the mixture at a temperature of about 20 to 40 °C for 30 minutes to 4 hours to prepare the compound represented by Chemical Formula 1-6. Preferably, the crystallization step may be carried out at room temperature of 20 to 30 °C, or 20 to 25 °C.

In this way, when a reaction product is prepared through crystallization using a crystallization solvent, unlike the case of purification using a silica column, it is possible to apply to industrialization, which is advantageous in terms of industrial mass production, and has the advantage of being able to obtain a high yield.

Specifically, the crystallization step can be carried out by crystallization using methyl isobutyl ketone or normal-heptane as a crystallization solvent. When both of the above solvents are used, the order is not limited, but it is preferable to proceed in the order of crystallization process using methyl isobutyl ketone followed by crystallization process using normal-heptane. The crystallization step can be carried out by repeating the crystallization process one or more times, which proceeds in the order of 'adding crystallization solvent and then stirring'. In this manner, when the crystallization process is repeated while adding the solvent multiple times instead of adding the solvent at once, it is possible to obtain even the crystals that adhere to the wall surface during the solvent addition process, thereby preventing a decrease in yield, and effectively removing by-products remaining after the reaction. Additionally, it is possible to crystallize an uncrystallized product through repeated crystallization processes. Thereby, compared with a process of purifying in a slurry form using a solvent such as ethyl acetate, a high-purity compound can be obtained in a high yield.

The crystallization solvent used in one crystallization process may be used in an amount of volume(mL/g) of 3 to 20 times, more specifically, in an amount of volume(mL/g) of 3 to 15 times, relative to the weight of the compound represented by Chemical Formula 1-5. One crystallization process may be carried out by stirring at a temperature range of 5 to 30 °C for 10 minutes to 3 hours. The process time can be appropriately adjusted, for example, by increasing it when the number of repetitions of the crystallization process is small, and decreasing it when the number of repetitions is large.

That is, when all the steps of purifying and crystallizing a reaction product of the step 3 are carried out to prepare the compound represented by Chemical Formula 1-6, the step 3 may include the following steps, but is not limited to these steps:
a step of reacting a compound represented by Chemical Formula 1-5 with an acid;
a step of adding water and dichloromethane to discard the organic layer (lower layer) and separate and extract the aqueous layer (upper layer), a step of adding water and ethyl acetate to discard the aqueous layer (lower layer) and separate the organic layer (upper layer), and a step of vacuum concentrating and/or azeotropically concentrating the purified mixture; and
a step of crystallizing the concentrate.

After crystallizing and purifying the compound represented by Chemical Formula 1-6, the compound can be dried at 30 to 60 °C for 10 to 14 hours to reduce the moisture content contained in the compound represented by Chemical Formula 1-6. More specifically, if the moisture content of the compound represented by Chemical Formula 1-6 can be significantly reduced by drying at 50 °C or less, the conversion rate of the next step (i.e., step 4 below) can be increased.

### (Step 4)

The step 4 is an acrylation reaction step in which the compound represented by Chemical Formula 1-6 and the compound represented by Chemical Formula 1-7 are reacted in the presence of a base to prepare the compound represented by Chemical Formula 1.

In the step 4, the compound represented by Chemical Formula 1-7 can be used in an amount of 0.1 to 10 equivalents based on 1 equivalent of the compound represented by Chemical Formula 1-6. Specifically, the compound represented by Chemical Formula 1-7 can be used in an amount of 0.2 to 5 equivalents, 0.5 to 5 equivalents, 0.5 to 3 equivalents, 0.5 to 2 equivalents, 0.5 to 1.5 equivalents, 1 to 1.5 equivalents, 1 to 1.1 equivalents, or 1.1 equivalents based on 1 equivalent of the compound represented by Chemical Formula 1-6.

As the base used in the reaction of the step 4, at least one selected from the group consisting of potassium carbonate, sodium hydroxide, lithium hydroxide, potassium hydroxide, triethylamine, diisopropylamine, diisopropylethylamine, sodium hydrogen carbonate, potassium hydrogen carbonate, cesium carbonate, sodium carbonate, sodium methylate and potassium butyrate may be used. Among these bases, it is preferable to use potassium carbonate, sodium carbonate, sodium hydrogen carbonate, or potassium hydrogen carbonate in terms of the completion of reaction and the formation of by-products.

A mixed solvent of tetrahydrofuran(THF) and water can be used as the solvent for this reaction. At this time, the tetrahydrofuran may be used in an amount of volume (mL/g) of 5 to 40 times, or 5 to 10 times, relative to the weight of the compound represented by Chemical Formula 1-6, and the water may be used in an amount of volume (mL/g) of 2 to 10 times, or 2 to 5 times, relative to the weight of the compound represented by Chemical Formula 1-6.

In addition, the reaction of the step 4 may be carried out at a temperature of -10 °C to 50 °C for 1 to 5 hours. Preferably, the reaction may be carried out at a temperature of 0 °C or more, more preferably at a temperature of 0 °C or more and 10 °C or less. Thereby, it is preferable that all of the reaction reagents, such as the reactants and organic solvents used to suppress the increase in the reaction temperature during the reaction are cooled to a temperature of 0 °C or less and then used.

Meanwhile, after the reaction of the step 4 is completed, the method may further comprise a purification step of extracting the reaction product. Specifically, the reaction product may be extracted using ethyl acetate and water. That is, the compound represented by Chemical Formula 1 may be extracted using ethyl acetate and water after the reaction is completed. The extraction using ethyl acetate and water may be carried out by a method in which the mixture containing the reaction product and each solvent are stirred for 30 minutes or more, allowed to stand for 30 minutes or more and then separated into layers, so that an aqueous layer (lower layer), is discarded and an organic layer (upper layer) is used. In the case of such an extraction, impurities having a relative retention time of less than 1.0 separated by high-performance liquid chromatography (HPLC) can be effectively removed into an aqueous layer, compared with the case of extraction using a methylene chloride/water mixed solvent. Additionally, if necessary, a purification process may be carried out using activated carbon and/or silica-based materials to remove by-products or foreign substances.

In addition, the step 4 may further comprise a step of filtering the organic layer containing the extracted mixture and then concentrating it under vacuum. This vacuum concentration step is preferably carried out after the extraction step using ethyl acetate and/or water.

Preferably, the step 4 may further comprise a step of crystallizing a reaction product in order to purify the compound represented by Chemical Formula 1. This crystallization step is preferably carried out after the extraction purification and vacuum concentration steps. In other words, the step 4 may be carried out by adding a crystallization solvent to the concentrated product after the extraction purification step and stirring it at a temperature of about 20 to 40 °C for 30 minutes to 4 hours. Preferably, the crystallization step may be carried out at room temperature of 20 to 30 °C, or 20 to 25 °C. The process time can be appropriately adjusted by increasing it when the number of repetitions of the crystallization process is small, and decreasing it when the number of repetitions is large.

When a reaction product is prepared through crystallization using a crystallization solvent in this manner, unlike the case of purification using a silica column, it is possible to apply to industrialization, which is advantageous in terms of industrial mass production, and has the advantage of being able to obtain a high yield.

Specifically, the crystallization step can be carried out by crystallization using ethyl alcohol or normal-heptane as a crystallization solvent. If both of the solvents are used, the order is not limited. The crystallization step can be carried out by repeating the crystallization process one or more times, which proceeds in the order of 'adding crystallization solvent and then stirring'. In this manner, when the crystallization process is repeated while adding the solvent multiple times instead of adding the solvent at once, it is possible to obtain even the crystals that adhere to the wall surface during the solvent addition process, thereby preventing a decrease in yield, and effectively removing by-products remaining after the reaction. Additionally, it is possible to crystallize an uncrystallized product through repeated crystallization processes. Thereby, compared with a process of purifying in a slurry form using a solvent, a high-purity compound can be obtained in a high yield.

The crystallization solvent may be used in an amount of volume (mL/g) of 0.5 to 15 times, more specifically, in an amount of volume (ml/g) of 0.5 to 10 times, relative to the weight of the compound represented by Chemical Formula 1-6. One crystallization process may be carried out by stirring at a temperature range of 5 to 30 °C, preferably at room temperature (20 to 25 °C) for 10 minutes to 2 hours. The progress time may be appropriately adjusted, for example, by increasing it when the number of repetitions of the crystallization process is small, and decreasing it when the number of repetitions is large.

Meanwhile, in order to crystallize more quickly, the crystal of the compound represented by Chemical Formula 1, which is the reaction product, may be added as a seed together with the solvent one or more times during the crystallization process. The seed may be added in an amount of 0.01 parts by weight or less based on 1 part by weight of the compound represented by Chemical Formula 1-6.

That is, when all the steps of purifying and crystallizing a reaction product of the step 4 is carried out in sequence to prepare the compound represented by Chemical Formula 1, the step 4 may include the following steps, but is not limited to these steps:
a step of reacting a compound represented by Chemical Formula 1-6 and a compound represented by Chemical Formula 1-7 in the presence of a base;
a step of adding ethyl acetate and water, discarding an aqueous layer (lower layer) and separating and extracting an organic layer (upper layer), and a step of vacuum concentrating the purified mixture; and
a step of crystallizing the concentrate.

After the compound represented by Chemical Formula 1 is crystallized, it can be dried at 30 to 60 °C for 8 to 14 hours to reduce the moisture content contained in the compound represented by Chemical Formula 1. More specifically, it can be dried at 50 °C or less.

### (Step 5)

The step 5 is a step of reacting a compound represented by chemical formula 1 with phosphoric acid to prepare a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

Specifically, the step can be carried out according to a preparation method comprising the following steps.
5-1) a step of dissolving 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one in an organic solvent;
5-2) a step of mixing phosphoric acid with the solution and stirring the mixture.

The step 5-2 is a step of preparing a salt in which 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one is chemically bonded with phosphoric acid. At this time, the salt in which 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one is chemically bonded with phosphoric acid may have a crystalline structure.

In the reaction, one or more organic solvents selected from the group consisting of methanol, ethanol, isopropanol, 1-butanol, 2-butanol, acetone, methyl ethyl ketone, ethyl acetate, methyl tert-butyl ether, acetonitrile, toluene, heptane, tetrahydrofuran, 3-methyltetrahydrofuran, 1,4-dioxane, dichloromethane, methyl isobutyl ketone, dimethylformamide(DMF), dimethylacetamide(DMAC), and dimethyl sulfoxide(DMSO) may be used. Such an organic solvent may be used in an amount of volume (mL/g) of 5 to 60 times, relative to the weight of the free base of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one. Preferably, the organic solvent may be used in an amount of volume (mL/g) of 10 to 40 times, relative to the weight of the free base of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

In the step 5, phosphoric acid can be used in an amount of 0.8 to 1.2 equivalents, preferably 1.0 to 1.2 equivalents, based on 1 equivalent of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

Next, the step of stirring the mixed solution of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, phosphoric acid and an organic solvent can be carried out at a temperature of 50 °C to 70 °C for 30 minutes to 30 hours.

The phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one prepared by the preparation method can be recovered from solution by a vacuum filtration process. If necessary, the recovered phosphate salt can be washed and vacuum dried to obtain a phosphate salt with high purity. In addition, reaction conditions such as the ratio of a solvent, temperature range, process time, and the like described in the above preparation methods may be adjusted according to the selected solvent.

### [Crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one]

The crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one may have three or more peaks selected from the group consisting of diffraction angles (2θ±0.2°) of 3.9°, 7.8°, 8.5°, 9.2°, 10.0°, 10.2°, 12.8 °, 14.4°, 15.8°, 16.0°, 20.8°, 21.2°, 21.6°, 22.3° and 25.5°in the X-ray powder diffraction pattern.

Further, the crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one may have an endothermic onset temperature of 106.52 ± 1.0 °C and shows a maximum endothermic peak at the endothermic temperature of 123.8 ± 1.0 °C in differential scanning calorimetry analysis.

When the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one or the crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one is used, the pK and solubility are improved and the stability is excellent, as compared with the case of using the free base.

Meanwhile, the present disclosure provides a pharmaceutical composition, comprising at least one selected from the group consisting of a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, and a crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

More specifically, the present disclosure provides a pharmaceutical composition for preventing or treating inflammatory diseases, autoimmune disease, proliferative disease, hyperproliferative disease, immunologically mediated disease, cancer, or tumor, comprising at least one selected from the group consisting of a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, and a crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

Such a pharmaceutical composition may include commonly used pharmaceutically acceptable carriers. The carriers are those usually used for the formulation, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyhydroxy benzoate, propylhydroxybenzoate, talc, magnesium stearate, mineral oil, and the like, but are not limited thereto. The pharmaceutical composition may further include a lubricant, a wetting agent, a sweetening agent, a flavoring agent, an emulsifying agent, a suspending agent, a preserving agent, and the like, in addition to the above ingredients.

The pharmaceutical composition may be administered in an oral mode, or in a parenteral mode including intravenous, intramuscular, intraperitoneal, subcutaneous and transdermal administration.

At this time, the pharmaceutical composition may be administered in a therapeutically effective dosage, for example, an effective dosage of about 0.001 mg/kg to about 100 mg/kg per day. The dosage can be changed according to the formulation method, administration mode, age, body weight, venereal condition, food, administration time, administration route, excretion rate or reaction sensitivity of the patient.

The pharmaceutical composition of the present disclosure may be formulated into a preparation by using a pharmaceutically acceptable carrier according to a method easily practicable by those skilled in the art, to which the present disclosure pertains, such that the composition may be prepared in a unit dose form or prepared by being inserted into a multi-dose container. At this time, the preparation can be used without limitation as long as it is in a form suitable for pharmaceutical preparations, including oral formulations such as powders, granules, tablets, capsules, suspensions, emulsions, syrups and aerosols, external preparations such as ointments and creams, suppositories, sterile injection solutions, and the like. The preparation may further include dispersants or stabilizers.

### [ADVANTAGEOUS EFFECTS]

A phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one and a crystalline form thereof according to the present disclosure have low hygroscopicity and improved stability and solubility as compared with the free base, and thus are suitable for pharmaceutical use. In addition, the method for preparing the phosphate slat or the crystalline form has the advantage that it can be mass-produced, and the safety of the preparation process is improved by using safer reactants than those used in conventional preparation methods.

### [BRIEF DESCRIPTION OF THE DRAWINGS]

FIG. 1 shows the results of NMR measurement of the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one prepared in Example 1.
FIG. 2 shows the X-ray powder diffraction pattern for the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one prepared in Example 1.
FIG. 3 shows the results of differential scanning calorimetry analysis of the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one prepared in Example 1.
FIG. 4 shows the results of thermogravimetric analysis of the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one prepared in Example 1.

### [DETAILED DESCRIPTION OF THE EMBODIMENTS]

Hereinafter, the present disclosure will be described in more detail with reference to Examples. However, the following Examples are for illustrative purposes only, and the scope of the present disclosure is not intended to be limited by the following Examples. In addition, in the following Examples, "volume" below can be understood to mean the ratio of the volume to the weight of the starting material in each step, "parts by weight" can be understood to mean the ratio of the weight relative to the starting material in each step, and "eq" can be understood to mean the equivalent of each material.

### Preparation Example: Preparation of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one (Compound represented by Chemical Formula 1)

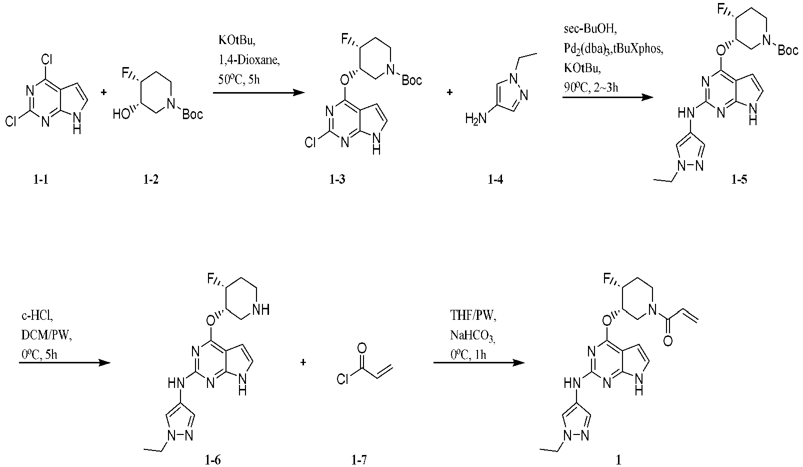

### Step 1) Preparation of Compound 1-3

2,4-Dichloro-7H-pyrrolo[2,3-d]pyrimidine (100.00 g, 1 eq) as Compound 1-1 and tert-butyl(3S,4R)-4-fluoro-3-hydroxypiperidine-1-carboxylate (122.45 g, 1.05 eq) as Compound 1-2 were dissolved in a mixture of 1000.0 mL(10 volume) of 1,4-dioxane and 10.0 mL(0.1 volume) of purified water, potassium tert-butoxide (179.05 g, 3 eq) was added thereto, and the mixture was stirred at 48 to 52 °C for 5 hours to complete the reaction.

Then, 1000.0 mL(10 volume) of ethyl acetate and 500.0 mL(5 volume) of purified water were added thereto, and the mixture was stirred for at least 30 minutes, allowed to stand for at least 30 minutes, and then separated into layers to discard an aqueous layer (lower layer). 500.0 mL(5 volume) of purified water was added to the remaining organic layer, and the mixture was stirred for at least 30 minutes, allowed to stand for at least 30 minutes and then separated into layers to discard an aqueous layer (lower layer). The remaining organic layer was vacuum concentrated at an external temperature of 50 °C or less, and 200.0 mL(2 volume) of 1,4-dioxane was added thereto, and the mixture was azeotropically concentrated at an external temperature of 50 °C or less.

200.0 mL(2 volume) of 1,4-dioxane was further added, and then the internal temperature was raised to 40~50°C to completely dissolve the mixture, and 400.0 mL(4 volume) of purified water was added and stirred at room temperature for at least 1 hour. Additionally, 600.0 mL(6 volume) of purified water was added thereto and stirred for at least 1 hour. The resulting mixture was filtered under reduced pressure using a filter, and washed with 500.0 mL(5 volume) of purified water. This was vacuum dried at an external temperature of 50 °C for 12 hours to obtain tert-butyl (3S,4R)-3-((6-chloro-1H-indol-4-yl)oxy)-4-fluoropiperidine-1-carboxylate (181.4 g, yield: 92.0%) as Compound 1-3.

1H NMR (500 MHz, CD₃OD): δ 7.206 (s, 1H), 6.552 (s, 1H), 5.461 (s, 1H), 5.093-4.997 (d, 1H), 3.908-3.793 (d, 2H), 3.559 (s, 2H), 2.210 (s, 1H), 1.926 (s, 1H), 1.287(s, 9H)

### Step 2) Preparation of Compound 1-5

Compound 1-3 (181.40 g, 1 eq) obtained from the step 1, 2-di-tert-butylphosphino-2',4',6'-triisopropylbiphenyl (8.31 g, 0.04 eq), tris(dibenzylideneacetone)dipalladium(0) (8.96 g, 0.02 eq) and potassium tert-butoxide (164.73 g, 3 eq) were added to 907.2 mL(5 volume) of sec-butyl alcohol, and then stirred at room temperature. Then, 1-ethyl-1H-pyrazol-4-amine (81.58 g, 1.5 eq) as Compound 1-4 was added, and then 181.4 mL(1 volume) of sec-butyl alcohol was added while washing the reactor walls and injection line so that all remaining reagents and raw materials would be used in the reaction. The resulting mixture was stirred at an internal temperature of 88 to 92 °C for 2 hours to complete the reaction.

Then, 907.2 mL(5 volume) of purified water and 907.2 mL(5 volume) of ethyl acetate were added thereto, and the mixture was filtered at an internal temperature of 50 °C, and washed with 907.2 mL(5 volume) of ethyl acetate. 907.2 mL(5 volume) of purified water was added once more to the filtrate, stirred for at least 30 minutes, allowed to stand for more than 30 minutes, and then separated into layers to discard an aqueous layer (lower layer). 907.2 mL(5 volume) of purified water was added to the remaining organic layer, stirred for at least 30 minutes, allowed to stand for at least 30 minutes, and then separated into layers to discard an aqueous layer (lower layer). 544.3 mL(3 volume) of 25 wt.% sodium chloride aqueous solution was added to the remaining organic layer, stirred for at least 30 minutes, allowed to stand for at least 30 minutes, and then separated into layers to discard an aqueous layer (lower layer). This was filtered and then washed with 907.2 mL(5 volume) of ethyl acetate. The filtrate was concentrated under vacuum at an external temperature of 60 °C or less, and then 544.3 mL(3 volume) of ethyl acetate was added. The mixture was then azeotropically concentrated at an external temperature of 60 °C or less to obtain tert-butyl (3S,4R)-3-((2-((1-ethyl-1H-pyrazol-4-yl)amino)-7H-pyrrolo[2,3-d]pyrimidin-4-yl)oxy)-4-fluoropiperidine-1-carboxylate (217.9 g, yield: 100.0%) as Compound 1-5. The obtained compound was used in the next step without purification.

### Step 3) Preparation of Compound 1-6

The concentrated residue of Compound 1-5 (217.9 g, 1 equivalents) obtained from the step 2 was added to and dissolved in 1090.0 mL(6 volume) of dichloromethane. The solution was cooled to 0 to 5 °C, and 414.2 mL(2.28 volume) of concentrated hydrochloric acid was slowly added so that the internal temperature did not exceed 5 °C, and then the mixture was stirred at 0 to 5 °C for 4 hours to complete the reaction.

Then, 1090.0 mL(6 volume) of dichloromethane and 1090.0 mL(6 volume) of purified water were added thereto, and the mixture was stirred for at least 30 minutes, allowed to stand for at least 30 minutes, and then separated into layers to discard an organic layer (lower layer). 1090.0 mL(6 volume) of dichloromethane was added to the remaining aqueous layer, and the mixture was stirred for 30 minutes or more, and allowed to stand for at least 30 minutes, and then separated into layers to discard an organic layer (lower layer). This was filtered, washed with 1090.0 mL(6 volume) of purified water, and 2179.9 mL(12 volume) of ethyl acetate was added and cooled to 0 to 5 °C. Then, about 1300.0 mL(7.17 volume) of 8N sodium hydroxide solution was added to adjust the pH to 9 to 11, allowed to stand for at least 30 minutes, and then separated into layers to discard an aqueous layer (lower layer). Additionally, 1090.0 mL(6 volume) of purified water was added, and the mixture was stirred for at least 30 minutes, allowed to stand for more than 30 minutes, and then separated into layers to discard an aqueous layer (lower layer). Vacuum concentration was then performed at an external temperature of 50 °C or less, 654.0 mL(3.6 volume) of methyl isobutyl ketone was added, and the mixture was azeotropically concentrated.

Additionally, 1090.0 mL(6 volume) of methyl isobutyl ketone was added, and the mixture was refluxed and stirred for at least 1 hour, and then the internal temperature was cooled to 20 to 25 °C, and 2179.9 mL(12 volume) of normal-heptane was slowly added and stirred for 2 hours. This was filtered and washed with 1090.0 mL(6 volume) of normal-heptane, and the washed filtrate was dried under vacuum at an external temperature of 50°C or less for 12 hours to obtain 126.75 g (2 step in-situ yield: 75.0%) of N-(1-ethyl-1H-pyrazol-4-yl)-4-(((3S,4R)-4-fluoropiperidin-3-yl)oxy)-7H-pyrrolo[2,3-d]pyrimidin-2-amine as Compound 1-6.

1H NMR (500 MHz, CD₃OD): δ 8.902 (s, 1H), 7.906 (s, 1H), 7.528(s, 1H), 6.932 (s, 1H), 6.277 (s, 1H), 5.375-5.330 (d, 1H), 5.166-5.066 (d, 1H), 4.104-4.061 (d, 2H), 3.011-3.002 (d, 2H), 2.826-2.664 (dd, 2H), 2.006-1.776 (m, 2H), 1.370 (s, 3H)

### Step 4) Preparation of Compound 1

Compound 1-6 (126.75 g, 1 eq, 1 weight part) obtained from the step 3, and sodium bicarbonate (61.66 g, 2 eq) were added to a mixed solution of 760.5 mL(6 volume) of tetrahydrofuran and 316.9 mL(2.5 volume) of purified water, and then cooled to 0 to 5 °C, and stirred for 1 hour. Acryloyl chloride (6.54 g, 1.1 eq) as Compound 1-7 was dissolved in 190.1 mL(1.5 volume) of tetrahydrofuran and slowly added so as not to exceed 5 °C. After the addition was completed, the mixture was stirred at 0 to 5 °C for 2 hours to complete the reaction.

Then, 1267.5 mL(10 volume) of purified water and 1267.5 mL(10 volume) of ethyl acetate were added thereto, and the mixture was stirred for at least 30 minutes, allowed to stand for at least 30 minutes, and then separated into layers to discard an aqueous layer (lower layer). This was filtered and washed with 380.3 mL(3 volume) of ethyl acetate. The filtrate was concentrated under vacuum at an external temperature of 50 °C or less, then 380.3 mL(3 volume) of ethyl alcohol was added thereto, and the mixture was concentrated under vacuum at an external temperature of 60 °C or less.

Additionally, 316.9 mL(2.5 volume) of ethyl alcohol was added, and then stirred at 20 to 25 °C to dissolve the mixture. 1.27 g (0.01 weight part) of Compound 1 as a seed was added at 20 to 25 °C, and stirred for 2 hours. When precipitation occurred sufficiently, 63.4 mL(0.5 volume) of ethyl alcohol was added, the fluidity was confirmed, followed by stirring for 2 hours. Then, 1140.7 mL(9 volume) of normal-heptane was added, and the mixture was stirred for 2 hours, and the crystallized liquid was filtered and then washed with 253.5 mL(2 volume) of normal-heptane. After washing, the filtrate was put in a dryer and vacuum-dried for 10 hours at an external temperature of 50°C or less to obtain 114.34 g (yield: 78%) of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one as Compound 1.

1H NMR (500 MHz, CD₃OD): δ 7.678 (s, 1H), 7.543-7.491 (d, 1H), 6.612-6.424 (m, 2H), 6.320-5.523 (m, 3H), 5.360-5.241 (m, 1H), 5.185-5.007 (m, 1H), 4.709-3.400 (m, 6H), 2.192-1.839 (m, 2H), 1.358-1.303 (m, 3H)

### Example 1: Preparation of a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one

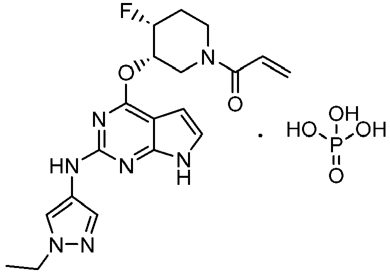

To 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one (10.0 g, 1 eq) prepared in Preparation Example, 100.0 mL(10 volume) of ethyl alcohol was added and dissolved by heating to an internal temperature of 60 to 65 °C. Foreign materials were filtered and transferred using a housing filter (0.45/0.22 µm), and then, the residue in the reactor was washed with 20 mL(2 volume) of ethyl alcohol, and the transfer was further carried out using a housing filter (0.45/0.22 µm). Then, the internal temperature was raised to 60-65 °C and the mixture was stirred.

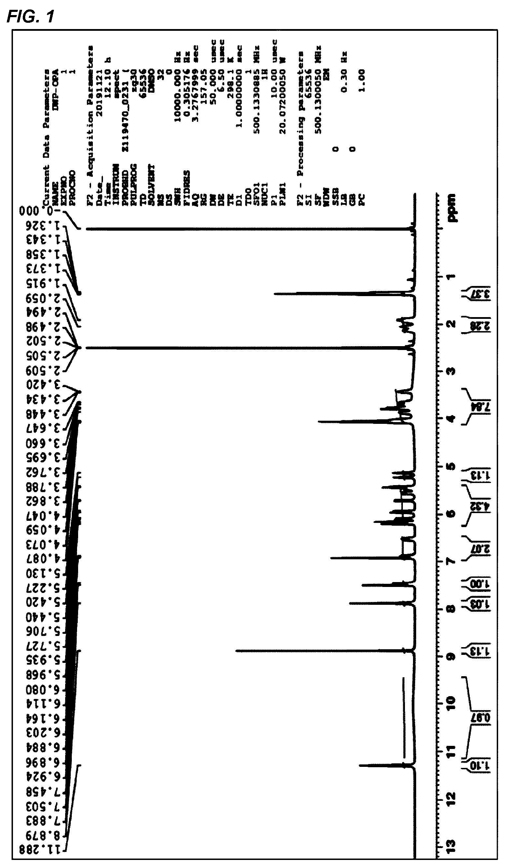

Phosphoric acid (4.8 g, 2 eq) was added to the reactor, stirred for 1 hour, and then concentrated at an external temperature of 50 to 55 °C. 30 mL of ethyl alcohol was added thereto and stirred for 2 hours, and then 100 mL of ethyl acetate was further added and stirred at room temperature (20-25 °C) for at least 2 hours. The reaction solution was cooled to -5-5 °C, stirred for at least 1 hour, then filtered, and washed with 30.0 mL(3 volume) of ethyl alcohol cooled to - 5~5 °C. The obtained filtrate was placed in a dryer and vacuum-dried at an external temperature of 55 °C or less to obtain 11.9 g (yield: 98%) of a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one. The results of NMR measurement of the obtained phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one are shown in FIG. 1. The phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one was obtained in a crystalline form.

1H NMR (500 MHz, DMSO-d₆) δ 7.89 (s, 1H), 7.52-7.48 (m, 1H), 6.93-6.50 (m, 2H), 6.29-5.45 (m, 4H), 5.33-5.13 (m, 1H), 4.10-3.42 (m, 6H), 2.28-1.85 (m, 2H), 1.37-1.32 (m, 3H)

### Experimental Example 1

### (1) X-ray powder diffraction pattern analysis of phosphate salt

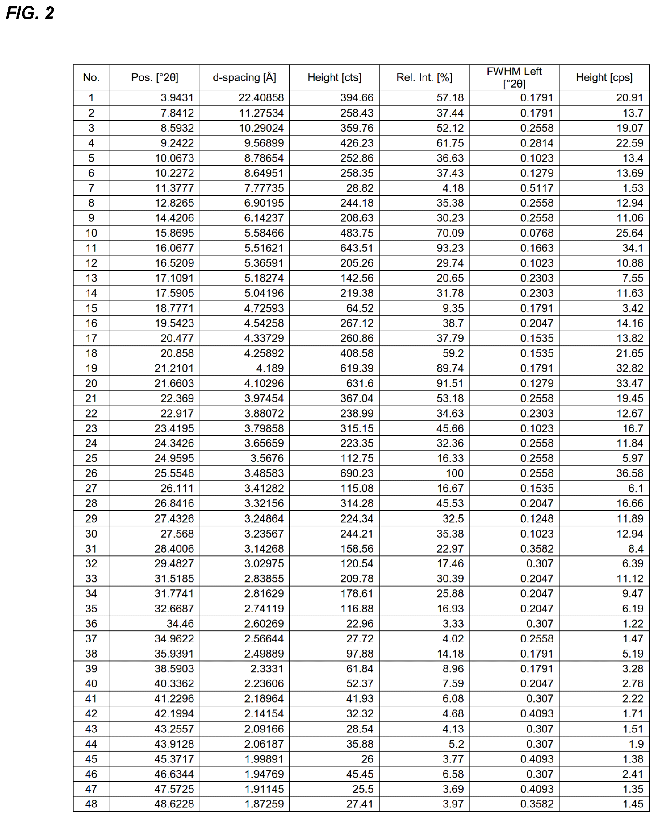

The phosphoric acid salt of Example 1 was subjected to X-ray powder diffraction analysis, and the results are shown in FIG. 2. At this time, the X-ray powder diffraction analysis was carried out using a PANalytical X' pert 3 powder x-ray diffractometer (Malvern Panalytical) by supplying CuK radiation (λ = 1.54056 Å) as the incident radiation to a copper anticathode tube (45 kV/40 mA). The step width was 0.02°, the measuring range was 4-50°, and the scan speed was 0.164129°/sec.

Referring to FIG. 2, it can be confirmed that the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one prepared in Example 1 has peaks at diffraction angles (2θ±0.2°) of 3.9°, 7.8°, 8.5°, 9.2°, 10.0°, 10.2°, 12.8 °, 14.4°, 15.8°, 16.0°, 20.8°, 21.2°, 21.6°, 22.3° and 25.5°in an X-ray powder diffraction pattern irradiated by Cu light source.

### (2) Differential scanning calorimetry and thermogravimetric analysis of phosphate salt

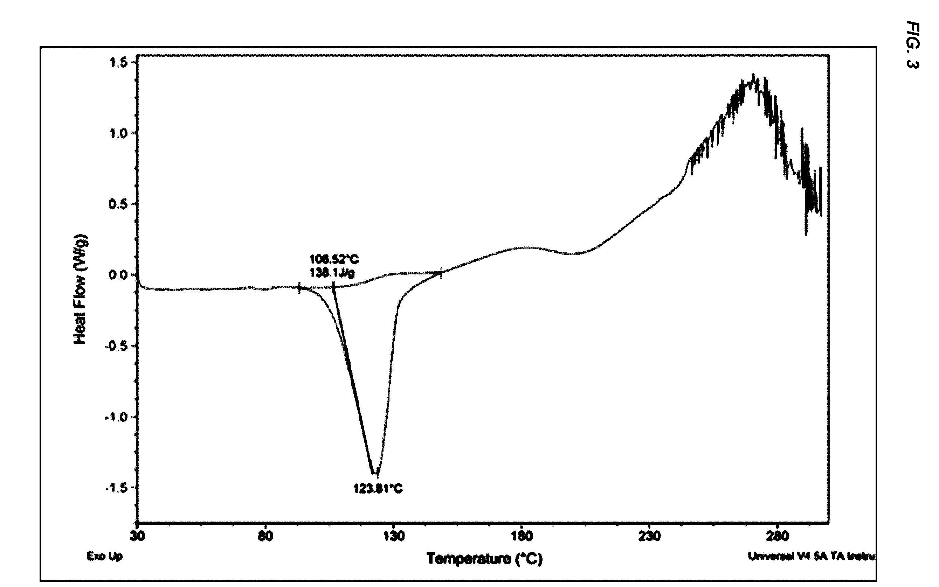

The phosphoric acid salt of Example 1 was subjected to differential scanning calorimetry analysis, and the results are shown in FIG. 3. At this time, the differential scanning calorimetry analysis was carried out using DSC Q2000 (available from TA Instruments). The sample was temperature programmed from 30°C to 300°C at a heating rate of 10°C/min, and 2 to 5 mg of the product was placed in a crimped aluminum capsule and analyzed.

Referring to FIG. 3, the phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one prepared in Example 2 has an endothermic onset temperature of 106.52 ±1.0 °C and shows a maximum endothermic peak at the endothermic temperature of 123.8 ±1.0 °C in differential scanning calorimetry analysis.

### (3) Comparison of the physical properties of the free base of Preparation Example and the phosphate salt of Example

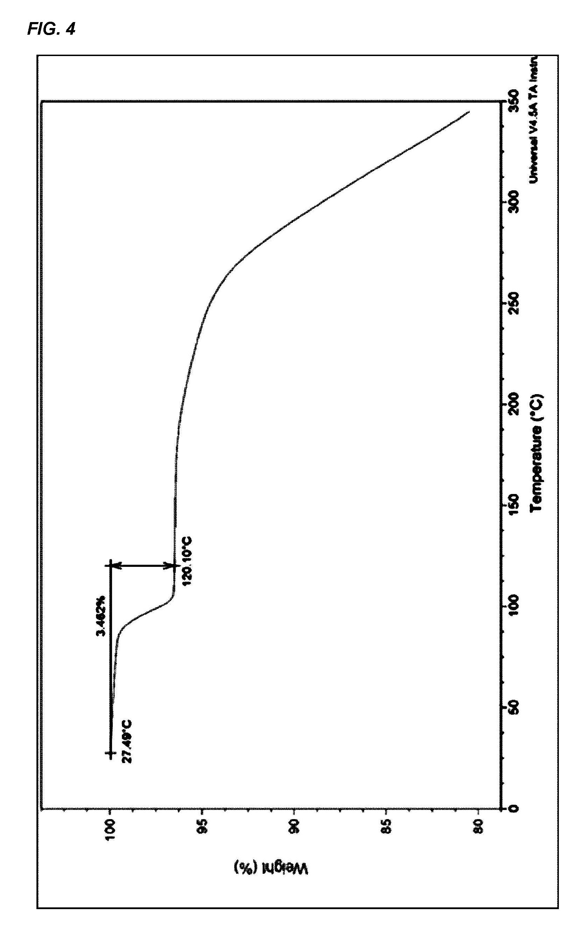

The physical properties of the free base (Compound 1) obtained in Preparation Example and the phosphate salt obtained in Example 1 were measured as follows.
1) Melting Point: Measured with MP70 available from Mettler Toledo. The sample was collected through a capillary, and then allowed to fall freely using a specified glass rod, and repeated several times to ensure that there was no empty space in the capillary tube. Then, the transmittance was measured starting from 168°C and increasing the temperature by 1°C per minute, and the melting point was confirmed. The measurement results are shown in Table 2 below.
2) Thermogravimetric analysis: Measured using TGA2 available from Mettler Toledo. 50 mg of the sample was placed in an Al₂O₃ pan, and the temperature was increased from 30.0°C at a rate of 10.00°C per minute to confirm the weight change up to 600.0 °C. Specific thermogravimetric analysis results are shown in FIG. 4.
3) Hygroscopicity analysis (DVS Hygroscopicity): Measured using DVS Adventure available from Surface Measurement Systems. The sample was exposed to 0~95% RH using DVS at 25°C to confirm the hygroscopicity. Depending on the degree of moisture adsorption, it was divided into Non-hygroscopic (0~0.12%)/Slightly Hygroscopic (0.2~2%)/Moderately Hygroscopic (2.0~15.0%)/Very Hygroscopic (>15.0%). The measurement results are shown in Table 2 below.
4) Water Solubility: Measured by HPLC available from Waters. The sample was supersaturated with water at 23~26°C, stirred for at least 18 hours, and then allowed to stand for at least 18 hours. The supernatant extracted using a centrifuge was filtered through a 0.45 µm filter. The HPLC area of the filtrate was measured to confirm the solubility. The measurement results are shown in Table 2 below.
5) Stability: The purity was measured after 2 weeks under the accelerated testing criteria of 40±2°C/75±5% relative humidity. About 1 mg of the phosphoric acid salt solid obtained in Example 1 was dissolved in 1 ml of 100% methanol, and then 700 µl of acetonitrile was added to 300 µl of this solution, diluted, and the purity was measured by HPLC. The measurement results are shown in Table 2 below.

The HLPC measurement conditions in 4) and 5) above are as follows.
- Detector: UV spectrophotometer (measurement wavelength: 230 nm)
- Diluent: 100% methanol
- Sample temperature: 15°C
- Injection volume: 15 µl
- Column: X Bridge C18 3.0 * 150 mm, 3.5 µm
- Column temperature: 30°C
- Analysis time: 40 min
- Flow rate: 0.6 ml/min
- Mode: Gradient
- Mobile phase A: 20 mM ammonium acetate
- Mobile phase B: 100% acetonitrile
- Mobile phase A and mobile phase B were controlled according to concentration gradient as shown in Table 1 below.

**[Table 1]**

| Time (min) | Mobile phase A(%) | Mobile phase B(%) |
|---|---|---|
| 0 | 75 | 25 |
| 1 | 75 | 25 |
| 18 | 60 | 40 |
| 30 | 20 | 80 |
| 33 | 20 | 80 |
| 37 | 75 | 25 |
| 40 | 75 | 25 |

**[Table 2]**

| | Free base of Preparation Example | Phosphate salt of Example |
|---|---|---|
| Crystallinity | Amorphous form | Crystalline form |
| Melting point | Tg 51 °C | 123°C |
| Hygroscopicity | Moderately Hygroscopic (~6%) | Moderately Hygroscopic (~3.4%) |
| Water solubility | 0.568 mg/mL | 2.388 mg/mL |
| Stability | 94.87% (2 weeks) | 99.50% (2 weeks) |

## Claims

1. A phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

2. The phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one according to claim 1,
wherein the phosphate salt has three or more peaks selected from the group consisting of diffraction angles (2θ±0.2°) of 3.9°, 7.8°, 8.5°, 9.2°, 10.0°, 10.2°, 12.8 °, 14.4°, 15.8°, 16.0°, 20.8°, 21.2°, 21.6°, 22.3° and 25.5° in an X-ray powder diffraction pattern irradiated by Cu light source.

3. The phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one according to claim 1,
wherein the phosphate salt has an endothermic onset temperature of 106.52 ± 1.0 °C and shows a maximum endothermic peak at the endothermic temperature of 123.8 ± 1.0 °C in differential scanning calorimetry analysis.

4. A method for preparing a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, the method comprising:
(step 1) reacting a compound represented by the following Chemical Formula 1-1 and a compound represented by the following Chemical Formula 1-2 in the presence of a base to prepare a compound represented by the following Chemical Formula 1-3;
(step 2) reacting a compound represented by the following Chemical Formula 1-3 and a compound represented by the following Chemical Formula 1-4 in the presence of a palladium catalyst and a base to prepare a compound represented by the following Chemical Formula 1-5;
(step 3) reacting a compound represented by the following Chemical Formula 1-5 with an acid to prepare a compound represented by the following Chemical Formula 1-6;
(step 4) reacting a compound represented by the following Chemical Formula 1-6 and a compound represented by the following Chemical Formula 1-7 in the presence of a base to prepare a compound represented by the following Chemical Formula 1; and
(step 5) reacting a compound represented by the following Chemical Formula 1 with phosphoric acid to prepare a phosphate salt of a compound represented by the following Chemical Formula 1,
wherein at least one of the steps 1 to 4 further comprises a crystallization step: 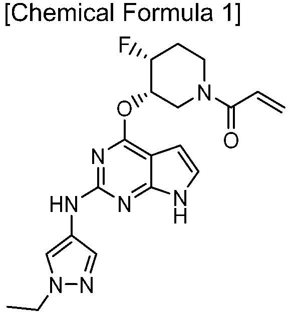 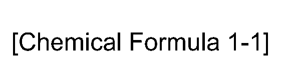 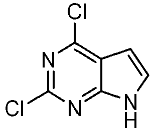 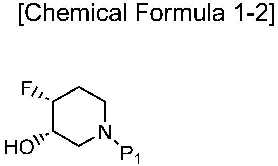 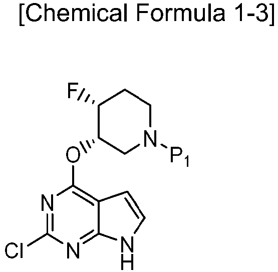 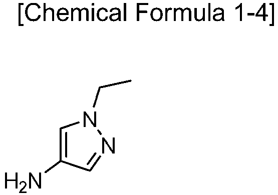 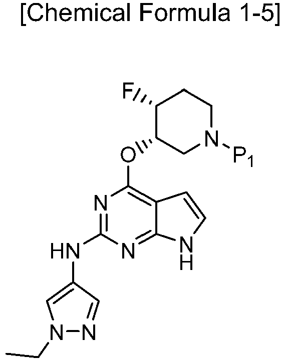 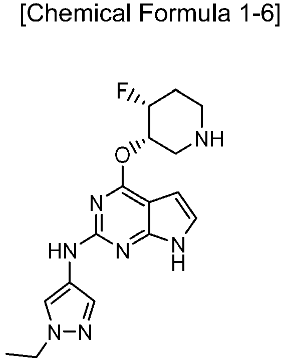 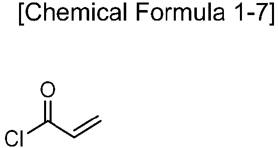 wherein in Chemical Formulas 1-1 to 1-7,
P₁ means a protecting group.

5. The preparation method according to claim 4,
wherein in the step 1, the compound represented by Chemical Formula 1-2 is used in an amount of 0.1 to 10 equivalents based on 1 equivalent of the compound represented by Chemical Formula 1-1.

6. The preparation method according to claim 4,
wherein in the step 1, the base is at least one selected from the group consisting of potassium tert-butoxide, sodium tert-butoxide, potassium methoxide, sodium methoxide, potassium tert-pentoxide, sodium tert-pentoxide, triethylamine, diisopropylethylamine, sodium hydroxide, potassium hydroxide, lithium hydroxide, potassium carbonate, calcium carbonate, sodium carbonate, potassium hydrogen carbonate, sodium hydrogen carbonate and cesium carbonate.

7. The preparation method according to claim 4,
wherein in the step 1, the base is used in an amount of 0.1 to 10 equivalents based on 1 equivalent of the compound represented by Chemical Formula 1-1.

8. The preparation method according to claim 4,
wherein in the step 1, the reaction is carried out in one or more organic solvents selected from the group consisting of tetrahydrofuran(THF), 1,4-dioxane, methanol, ethanol, isopropanol, 1-butanol, 2-butanol, acetone, methyl ethyl ketone, ethyl acetate, methyl tert-butyl ether, acetonitrile, toluene, heptane, 3-methyltetrahydrofuran, dichloromethane, methyl isobutyl ketone, dimethyl formamide(DMF), dimethyl acetamide(DMAC), and dimethyl sulfoxide(DMSO).

9. The preparation method according to claim 8,
wherein in the step 1, the reaction is carried out at 30 to 60 °C.

10. The preparation method according to claim 4,
wherein the step 1 further comprises a step of crystallizing a reaction product after reacting the compound represented by Chemical Formula 1-1 and the compound represented by Chemical Formula 1-2 in the presence of a base.

11. The preparation method according to claim 4,
wherein in the step 2, the compound represented by Chemical Formula 1-4 is used in an amount of 0.1 to 10 equivalents based on 1 equivalent of the compound represented by Chemical Formula 1-3.

12. The preparation method according to claim 4,
wherein in the step 2, the palladium catalyst is at least one selected from the group consisting of tris(dibenzylideneacetone)dipalladium(0), tetrakis(triphenylphosphine)palladium(0), bis[tris(2-methylphenyl)phosphine]palladium, palladium(II)acetate, 1,1-(bis(diphenylphosphino)ferrocene)palladium(II)dichloride, and palladium(II)chloride.

13. The preparation method according to claim 4,
wherein in the step 2, the base is at least one selected from the group consisting of potassium tert-butoxide, sodium tert-butoxide, potassium methoxide, sodium methoxide, potassium tert-pentoxide, sodium tert-pentoxide, triethylamine, diisopropylethylamine, sodium hydroxide, potassium hydroxide, lithium hydroxide, potassium carbonate, calcium carbonate, sodium carbonate, potassium hydrogen carbonate, sodium hydrogen carbonate and cesium carbonate.

14. The preparation method according to claim 4,
wherein in the step 2, the reaction is carried out in one or more organic solvents selected from the group consisting of tetrahydrofuran(THF), 1,4-dioxane, sec-butyl alcohol, methanol, ethanol, isopropanol, 1-butanol, 2-butanol, tert-butyl alcohol, acetone, methyl ethyl ketone, ethyl acetate, methyl tert-butyl ether, acetonitrile, toluene, xylene, heptane, 3-methyltetrahydrofuran, dichloromethane, methyl isobutyl ketone, dimethyl formamide(DMF), dimethyl acetamide(DMAC), and dimethyl sulfoxide(DMSO).

15. The preparation method according to claim 4,
wherein the reaction of the step 2 is carried out at 80 to 100 °C.

16. The preparation method according to claim 4,
wherein in the step 3, the acid is at least one selected from the group consisting of hydrochloric acid, acetic acid, trichloroacetic acid, trifluoroacetic acid, bromic acid, sulfuric acid, nitric acid and iodic acid.

17. The preparation method according to claim 4,
wherein in the step 3, the reaction is carried out in one or more organic solvents selected from the group consisting of ethyl acetate, dichloromethane, butyl acetate, trichloromethane, methanol, ethanol, isopropanol, 1-butanol, 2-butanol, acetone, methyl ethyl ketone, methyl tert-butyl ether, acetonitrile, toluene, heptane, tetrahydrofuran, 3-methyltetrahydrofuran, 1,4-dioxane, methyl isobutyl ketone, dimethylformamide (DMF), dimethylacetamide (DMAC), and dimethyl sulfoxide (DMSO).

18. The preparation method according to claim 4,
wherein the reaction of the step 3 is carried out at 0 °C to 50 °C.

19. The preparation method according to claim 4,
wherein the step 3 further comprises a step of crystallizing a reaction product after reacting the compound represented by Chemical Formula 1-5 with an acid.

20. The preparation method according to claim 4,
wherein in the step 4, the compound represented by Chemical Formula 1-7 is used in an amount of 0.1 to 10 equivalents based on 1 equivalent of the compound represented by Chemical Formula 1-6.

21. The preparation method according to claim 4,
wherein in the step 4, the base is at least one selected from the group consisting of potassium carbonate, sodium hydroxide, lithium hydroxide, potassium hydroxide, triethylamine, diisopropylamine, diisopropylethylamine, sodium hydrogen carbonate, potassium hydrogen carbonate, cesium carbonate, sodium carbonate, sodium methylate and potassium butyrate.

22. The preparation method according to claim 4,
wherein the reaction of the step 4 is carried out at -10 °C to 50 °C.

23. The preparation method according to claim 4,
wherein the step 4 further comprises a step of crystallizing a reaction product after reacting the compound represented by Chemical Formula 1-6 and the compound represented by Chemical Formula 1-7 in the presence of a base.

24. The preparation method according to claim 4,
wherein in the step 5, the reaction is carried out in one or more organic solvents selected from the group consisting of 2-butanol, acetone, methyl ethyl ketone, ethyl acetate, methyl tert-butyl ether, acetonitrile, toluene, heptane, tetrahydrofuran, 3-methyltetrahydrofuran, 1,4-dioxane, dichloromethane, methyl isobutyl ketone, dimethylformamide (DMF), dimethylacetamide (DMAC), and dimethyl sulfoxide (DMSO).

25. A crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

26. The crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one according to claim 25,
having has three or more peaks selected from the group consisting of diffraction angles (2θ±0.2°) of 3.9°, 7.8°, 8.5°, 9.2°, 10.0°, 10.2°, 12.8 °, 14.4°, 15.8°, 16.0°, 20.8°, 21.2°, 21.6°, 22.3° and 25.5°in an X-ray powder diffraction pattern.

27. The crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one according to claim 25,
having an endothermic onset temperature of 106.52 ± 1.0 °C and shows a maximum endothermic peak at the endothermic temperature of 123.8 ± 1.0 °C in differential scanning calorimetry analysis.

28. A pharmaceutical composition for preventing or treating inflammatory diseases, autoimmune disease, proliferative disease, hyperproliferative disease, immunologically mediated disease, cancer, or tumor, comprising at least one selected from the group consisting of a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, and a crystalline form of phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

29. A method for preparing a phosphate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one,
comprising a compound represented by the following Chemical Formula 1-5 as an intermediate: 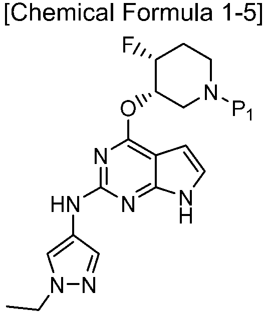 wherein in Chemical Formula 1-5,
P₁ means a protecting group.

30. The preparation method according to claim 29,
wherein P₁ is tert-butoxycarbonyl (Boc).

31. A compound represented by the following Chemical Formula 1-5: 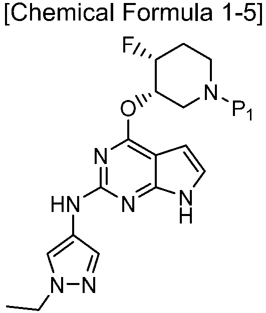 wherein in Chemical Formula 1-5,
P₁ means a protecting group.

32. The compound according to claim 31,
wherein P₁ is tert-butoxycarbonyl (Boc).
